# EUROPEAN PATENT SPECIFICATION

(11) **EP 3 132 023 B1**
(45) Date of publication and mention of the grant of the patent: **02.06.2021**
(21) Application number: 15723435.2
(22) Date of filing: 15.04.2015
(51) Int. Cl.: C12N 5/00, C12N 5/071

(54) **METHOD OF PREPARING CELLS FOR 3D TISSUE CULTURE**
VERFAHREN ZUR HERSTELLUNG VON ZELLEN FÜR 3D-GEWEBEKULTUR
PROCÉDÉ DE PRÉPARATION DE CELLULES POUR UNE CULTURE DE TISSU 3D

(30) Priority: 15.04.2014 GB 201406716
(43) Date of publication of application: 22.02.2017
(73) Proprietor: Insphero AG, 8952 Schlieren (CH)
(72) Inventor: MESSNER, Simon, 8049 Zürich (CH); MORITZ, Wolfgang, 5454 Bellikon (CH); LICHTENBERG, Jan, 8103 Unterengstringen (CH); KELM, Jens M., 8610 Uster (CH)
(74) Representative: Michalski Hüttermann & Partner Patentanwälte mbB
(86) International application number: PCT/EP2015/058174
(87) International publication number: WO 2015/158777

(56) References cited:
- WO-A1-2006/032092
- WO-A1-2008/100497
- WO-A1-2010/060080
- WO-A1-2011/154552
- WO-A1-2012/083023
- WO-A2-2007/124023
- WO-A2-2008/051568
- WO-A2-2011/064669
- WO-A2-2013/040078
- US-A1- 2010 124 569
- US-A1- 2010 129 330
- US-A1- 2011 229 970
- US-A1- 2012 276 068
- MARTIN I ET AL: "Enhanced cartilage tissue engineering by sequential exposure of chondrocytes to FGF-2 during 2D expansion and BMP-2 during 3D cultivation", JOURNAL OF CELLULAR BIOCHEMISTRY, WILEY-LISS INC, US, vol. 83, no. 1, 1 January 2001 (2001-01-01), pages 121-128, XP002265721, ISSN: 0730-2312, DOI: 10.1002/JCB.1203

## Description

### Field of the invention

The present invention relates to the field of 3D tissue cultures.

### Introduction

A 3D (three dimensional) tissue culture is an artificially-created environment in which biological cells are permitted to grow or interact with its surrounding environment in all three dimensions.

3D cultures are an improvement over 2D (two dimensional) cultures (also called "monolayers") for many reasons. In living tissues, cells exist in 3D microenvironments with intricate cell-cell and cell-matrix interactions and complex transport dynamics for nutrients and cells. Standard 2D, or monolayer, cell cultures are inadequate representations of this environment.

The main applications for 3D tissue culture lie in drug efficacy and/or toxicity screenings, investigative/mechanistic toxicology, target discovery/identification, drug repositioning studies, and pharmacokinetics and pharmacodynamics assays, although other applications are also available, like for regenerative medicine.

With respect to the former, inadequate 2D environment often makes 2D cultures unreliable predictors of *in vivo* drug efficacy and toxicity. 3D constructs more closely resemble in vivo tissue in terms of cellular communication, the formation of biochemical and physico-chemical gradients and the development of extracellular matrices.

These matrices help the cells to move within the 3D construct similar to the way cells would move in living tissue. 3D constructs are thus improved models for cell migration, differentiation, survival, and growth. Furthermore, 3D tissue cultures provide more accurate depiction of cell polarization, since in 2D, the cells can only be partially polarized. The latter is particularly important for hepatocytes, which are one of the preferred cell types in 3D tissue cultures used for drug efficacy and toxicity screenings. Moreover, cells grown in 3D exhibit different gene expression than those grown in 2D (Kelm et al 2010, Ridky et al 2010, Cody et. al. 2008).

A real 3D environment is often necessary for cells in vitro to form important physiological structures and functions. The third dimension of cell growth provides more contact space for mechanical inputs and for cell adhesion, which is necessary for integrin ligation, cell contraction and even intracellular signaling. Normal solute diffusion and binding to effector proteins (like growth factors and enzymes) is also reliant on the 3D cellular matrix, so it is critical for the establishment of tissue scale solute concentration gradients.

Because of the fact that cellular interactions are key for the basic function and sustainability of a 3D tissue, the quality of the cells used is of paramount importance. Cells with minor quality do not integrate into such tissue, or integrate only partially preventing the nascending tissue to adapt organotypic architecture. This results in dysfunctional regions which would be integral part of the tissue, and eventually the entire tissue would be affected in terms of morphology, functionality, viability or stability.

The state of the art provides cell viability assays which yields information on the viability of cells used in a culture.

However, such assays provide an overall signal on cell viability only, but do not provide any information on the viability of individual cells. For these reasons, cell viability assays do not enable the separation of unsuitable cells and thus cannot be discarded which, due to lack of viability or vitality, would negatively interfere with the 3D tissue culture process.

US 2012/276068 A1 discloses a method for treating a degenerative or traumatic injury to a nerve tissue or the brain by administering a composition containing adherent bone marrow stem cells, which are obtained from a population of isolated bone marrow cells. WO 2013/040078 relates to living, three-dimensional tissue constructs for in vitro scientific and medical research, arrays thereof, and methods of making the tissues and arrays.

### Summary of the invention

It is thus one object of the present invention to provide an approach to overcome the above problems.

It is another object of the present invention to provide an approach suitable for 3D tissue culture which helps to identify cells of minor quality or viability prior to formation of the tissue.

It is another object of the present invention to provide an approach suitable for 3D tissue culture which helps to sort out cells of minor quality or viability prior to formation of the tissue.

It is another object of the present invention to provide an approach suitable for 3D tissue culture which helps improves the functionality, viability and stability of 3D tissues.

These objects are achieved by the subject matter of the independent claims, while the dependent claims as well as the specification disclose further preferred embodiments.

### Embodiments of the invention

The present invention is defined by the method of preparing cells for 3D tissue culture according to appended claim 1.

According to one example of the disclosure, a method of preparing cells for 3D tissue culture is provided, which method comprises the steps of
a) plating the cells on a suitable surface
b) optionally, checking for their capability to adhere to said surface,
c) discarding the cells which have not adhered to said surface, and
d) detaching the adhered cells and transferring them into a 3D tissue culture process.

The said process is also called "vital cell selection". According to methods from the prior art, cell viability (live or dead) is often assessed prior to transferring cells into a 3D tissue culture process. This can be done, for example by means of suitable viability assays, which determine the ability of cells to maintain or recover their viability. A number of methods are available for this purpose. For example, the trypan blue test, which is a membrane leakage assay, tests whether cells are permeable to trypan blue. If yes, they are considered non-viable. Cell viability is for example calculated as the number of viable cells divided by the total number of cells within the grids of a hemocytometer, onto which a subsample of the cells that are meant to be transferred into a 3D tissue culture process is placed.

Thus, the respective test provides an overall signal on cell viability, which, although it has been obtained by assessment of individual cells, does not provide any information on the viability of individual cells which are meant to be transferred to the, 3D tissue culture process, mainly because the cells which are actually subjected to said test are discarded later on, and are not being further used for the 3D tissue culture, because they have been treated with the testing agent. Thus, the viability assay is in most cases performed with a sample of cells which is meant to be representative for the cells that are used for the 3D tissue culture process.

Therefore, viability tests do not allow the separation of dead or unsuitable cells from the total cell pool which is meant to be used for the 3-D tissue culture process. They only provide information about the overall viability of the cells which are meant to be used.

However, although such information may be helpful, it does not help to discard those cells which, due to lack of viability or vitality, would negatively interfere with the 3D tissue culture process. Because a 3D tissue is characterized by (i) a high density of cells, (ii) the development of an extracellular matrix, and (iii) the establishment of cell-cell contacts, the presence of nonviable cells and cells with a low vitality can seriously affect the function of a 3D tissue, with the ultimate effect that the 3D tissue becomes dysfunctional in case the amount of nonviable cells in the tissue is too high.

Further, cells which are not capable of integrating into a 3D tissue, probably because they can no longer establish cell-cell contacts, may still pass the viability test, for example because their membrane is still intact and thus impermeable to the test reagent, e.g. trypan blue.

As an alternative, cells may be sorted through FACS prior to using them, in order to separate viable from non-viable cells, e.g., on the basis of a dual fluorescent CalceinAM/PI approach.

Further, the inventors have found that, while such FACS assay delivers a cell-specific information which helps to sort out viable from non-viable cells, the group classified as "viable" encompasses cells would not be fully functional in a 3D tissue. Thus, the latter group forms a subgroup of all viable cells, which can not be sorted out by the above techniques.

The method according to the invention solves this problem by providing a tool which allows an efficient selection of those cells which are viable enough that they can be used for forming a 3D tissue, while it further allows to discard those cells which lack such viability.

The standard applied by the method according to the invention relies on the plateability of the cells, i.e., on their capability to adhere to a cell culture surface, which again is an excellent measure for their viability, and for their suitability to integrate into a 3D network of cells as it occurs in 3D tissue culture. The plateability depends on the cell's capability to form an extracellular matrix, which in turn is a feature that is vital for their integration into a 3D network of cells.

Plating cells, however, is a standard approach that is mainly used in 2D cell culture, not in 3D cell culture. In 3D cell culture, such step is apparently useless, because it adds further effort to a cell culturing method, which as such is subject of many uncertainties anyway, and it subjects the cells to additional stress, with the risk that cells get lost, or lose viability, which otherwise could still be used in 3D cell culture.

In this context it is important to understand that the provision of sufficient cell mass is a critical issue in 3D cell culture; not only in those cases where 3D cell culture is meant to be used for regenerative medicine purposes, but also in cases where 3D cell culture is meant to be used for screening purposes, e.g., drug screening and the like. For this reason, the skilled person is extremely cautious not to lose cell mass during the handling and preparation of cells for 3D cell culture.

This scrutiny is even fostered by the fact that in many cases, the cells used for 3D cell culture are primary human cells that are derived from organ donations. Generally, organs are used which do not qualify for therapeutic transplantation. However even these organs are in extremely short supply. For this reason, the skilled person is inclined not to put cells at risk during handling and preparation for 3D culture.

The inventors of the present invention were aware of this risk, and the related prejudice. However, they found out, surprisingly, that the advantage that is obtained when using the method according to the invention compensates for the above-mentioned disadvantages.

In other words, they found out that the increased quality and functionality of the 3D tissues that can be obtained when dysfunctional cells are sorted out in a 2D plating step prior to the 3D culture process, justifies a potential loss of cell mass during this step.

In one experimental approach, e.g., the inventors used hepatocytes which were obtained from a human donor liver by means of collagenase digestion, as disclosed in Pichard et al. (2006). This process yielded up to 300 vials with up to 8 x 10⁶ hepatocytes each. The plating step that is implemented according to the invention prior to the 3D tissue fabrication can causes a loss of up to 60 % of the cells, i.e., 4.8 out of 8 million cells get lost because they do not adhere sufficiently to the plate surface, and 3.2 million hepatocytes are retained, which can then be used for the 3D tissue fabrication, providing up to 3000 microtissues of superior quality.

The plating process thus results in a loss of cells, but helps to improve the quality of the 3D tissues, which otherwise would have been wasted due to the negative interference of cells with low vitality.

It is important to understand that the step of plating the cells and discarding those cells which have not adhered, as encompassed by the subject invention, is not a viability assay according to the state-of-the-art. In *strictu sensu,* it is not an assay at all, because, in its broadest sense, it simply involves a plating step and a subsequent washing step to remove the cells that have not adhered.

In a preferred embodiment, the plating step as such may last anything between 30 mins und 98 hrs. Further details will be discussed elsewhere herein.

According to the method of the invention, it does not encompass the application of a viability assay throughout steps a) - d).

As discussed above such viability assay (i) provides an overall signal on cell viability only, (ii) does not provide any information on the viability of individual cells, (iii) does not allow the separation of unsuitable cells and (iv) does not help to discard those cells which, due to lack of viability or vitality, would deteriorate the 3D tissue culture process.

Thus, although such test would not be harmful, it's informative value is very limited in the current context and would not serve the purpose of clearing the cell population from unsuitable cells.

According to a preferred embodiment of the invention, the 3D tissue culture process is at least one selected from the group consisting of
- scaffold-based 3D tissue culture
- hydrogel-based 3D tissue culture
- cellular self-assembly 3D tissue culture
- 3D bioprinting,

Preferred examples for cellular self-assembly are hanging drop cultures, liquid overlay cultures or cultures on micro-patterned surfaces.

The hanging-drop technique was initially developed for cultivating stem cell embryoid bodies. To prepare a hanging-drop culture, cell suspension drops are suspended at the lower side of a suitable plate. Preferably, so-called hanging drop plates are used for this purpose which resemble microtiter plates, and come in similar formats, but have holes through which the cell suspension liquid can be delivered. Such type of system is available from InSphero AG, Switzerland, under the brand name "GravityPLUS™ 3D Culture and Assay Platform." The hanging drops can assume volumes of between 2 and 100 µl, and accommodate between 100 and 50000 cells which then are meant to form the 3D tissue. In each drop the cells are concentrates at the bottom of the drop simply by gravity, which then form single spheroids at the air-liquid interface. Background and protocols and are disclosed in Kelm et al, 2003, and Kelm & Fussenegger 2004.

The hanging-drop technique allows cells to form a tissue which in other vessels would tend to adhere to a solid-liquid interface, like the flat plane of culture dishes. A hanging drop is devoid of such interfaces, thus allowing cells to form a 3D construct that resembles, in its structure, a true tissue.

The liquid overlay culture technique is another technique to form 3D tissue spheroids. In this approach, a multititer plate is made non adherent, e.g., by coating with agarose or by photodynamic chemical patterning. Cells are then seeded in the wells at a density of 100 - 10000 cells per well and are then incubated in culture medium. Protocols are disclosed, e.g., in Yuhas et al (1977) or Metzger et al (2011).

3D bio-printing is a process in which cells are plotted on a matrix where they form a tissue. 3D bio-printing may involve techniques like photolithography, magnetic bioprinting, stereolithography, and cell extrusion.

Scaffold-based 3D tissue culture uses a scaffold on which cells are seeded. The scaffold may, or may not, be biodegradeable (e.g., chitosan, polylactic acid, polystyrene, etc). A broad discussion on this technique can be found in Carletti et al (2011).

Hydrogel-based 3D tissue culture (also called "scaffoldless 3D tissue") is an approach were cells are grown in a hydrogel matrix and form a 3D tissue. This technique is for example disclosed in Geckil et al. (2010).

It is important to understand that the method of the present invention can be advantageously used in all of the above approaches. One key feature of the present invention is that non-viable cells are sorted out prior to the culture process. Therefore, the quality of the resulting tissue increases significantly, because dysfunctional cells which could disturb the tissue-forming process have been removed. This advantage does not only apply to the hanging drop technique, for which examples are shown herein, but also for scaffold-based 3D tissue culture, hydrogel-based 3D tissue culture, 3D bioprinting, and other cellular self-assembly 3D tissue culture methods, like liquid overlay.

According to a particularly preferred embodiment of the invention, the cells are crypopreserved cells which are thawed before plating.

Most of the cell types discussed herein may be cryopreserved, and can still be used after thawing thereof. The potential of cryopreservation is important to have sufficient cell mass on stock for on demand production capacities of 3D tissues of a given kind.

Likewise preferred, however, the cells are non-frozen cells. This approach allows on-the-fly production of 3D tissues if desired so, but requires the availability of unfrozen, viable cells, e.g., obtained directly from a donor organ.

According to a particularly preferred embodiment of the invention, the cells used are selected from
- primary cells
- stem cells, and/or
- immortalized cells.

Cells that are cultured directly from a subject are known as primary cells. With the exception of some derived from tumors, most primary cell cultures have limited lifespan. After a certain number of population doublings (called the Hayflick limit), cells undergo the process of senescence and stop dividing, while generally retaining viability.

Stem cells are undifferentiated, or partly differentiated, biological cells that can differentiate into specialized cells and can divide (through mitosis) to produce more stem cells. They are found in multicellular organisms. In mammals, there are two broad types of stem cells: embryonic stem cells, which are isolated from the inner cell mass of blastocysts, and adult stem cells, which are found in various tissues. In adult organisms, stem cells and progenitor cells act as a repair system for the body, replenishing adult tissues. The use of human pluripotent stem cells (hPSC) in the development of 3D tissue models based has been described by Jensen et al (2009). Sartipy & Björquist (2011) describe the use of hPSC-Based Models for Cardiac and Hepatic Toxicity Assessment.

An immortalized cell line is a population of cells from a multicellular organism which would normally not proliferate indefinitely but, due to mutation, have evaded normal cellular senescence and instead can keep undergoing division. The cells can therefore be grown for prolonged periods in vitro. The mutations required for immortality can occur naturally or be intentionally induced for experimental purposes. Immortal cell lines are a very important tool for research into the biochemistry and cell biology of multicellular organisms. One example for such cell line are HepaRG cells, which are terminally differentiated hepatic cells derived from a human hepatic progenitor cell line that retains many characteristics of primary human hepatocytes.

According to the invention, the cells are not expanded prior to, during or after plating, and/or the cells are not passaged after plating.

While a couple of primary cells, like Fibroblasts or Chondrocytes, can be expanded in culture, most primary cells have only a limited capacity of being expanded. In contrast thereto, stem cells, immortalized cells and tumor cells have, generally speaking, the capacity of being expanded.

Plating of cells plays a role only in one particular subdivision of 3D cell culture, namely in those types of cell culture where cells are expanded prior to transferring them into a 3D tissue culture process. As discussed above this applies only to a very limited number of primary cells, as well as to stem cells, tumor cells and immortalized cells.

Further, expansion requires serial passaging of the cells, which is performed mainly in monolayer cultures such as T-flasks or Roller bottles later in the process. One major drawback of cell expansion is potential loss of the native cell phenotype (de-differentiation), which is followed by a loss of native functionality, and genetic/chromosomal alterations. Some of the most important cell types, in particular for toxicity assays and for regenerative medicine, cannot be expanded anyway.

According to another preferred embodiment of the invention, the cells are mammalian cells, preferably selected from the group consisting of:
- humane cells
- cynomolgus cells
- pig cells
- canine cells, and/or
- rat cells.

It is particularly preferred that the cells used are Hepatocytes, Hepatocytes are cells of the main tissue of the liver, which make up 70-85% of the liver's cytoplasmic mass. Primary cultures of human hepatocytes are an *in vitro* model widely used to investigate numerous aspects of liver physiology and pathology, as well as for screening purposes, e.g., toxicity assays and the like. The technique used to isolate human hepatocytes is based on a two-step collagenase perfusion of a donated liver.

The functionality of hepatocytes is highly dependent on their capability of forming a polar phenotype. This does not only affect their usability for screening purposes, but also their life span, which under sub optimal conditions does rarely exceed 5 days. Such polar phenotype, however, is only established in 3D culture, which mimics the cell's natural environment, but not in 2D culture. 3D tissues made from hepatocytes are thus often nicknamed "organ on a chip or "organ in a well".

According to another aspect of the disclosure, a 3D tissue culture process is provided, said process being selected from the group consisting of
- scaffold-based 3D tissue culture
- hydrogel-based 3D tissue culture
- cellular self-assembly 3D tissue culture, and/or
- 3D bioprinting
and wherein, in said process, cells are used that have been prepared according to the method according to the invention.

According to another aspect of the disclosure, a 3D tissue culture is provided that has been obtained with the above process and/or from cells that have been prepared according to the method of the invention.

Preferably, said which 3D tissue culture adopts the shape of a spheroid, which assumes preferably, a volume of between ≥ 2 and ≤ 500 µl, and accommodates, preferably, between ≥ 100 and ≤ 50000 cells which then are meant to form the 3D tissue.

According to still another aspect of the invention, the use of a 3D tissue according to the invention for at least one purpose selected from the group consisting of
- drug efficacy and/or toxicity screenings,
- investigative/mechanistic toxicology,
- target discovery/identification,
- drug repositioning studies,
- pharmacokinetics and pharmacodynamics assays, and/or
- regenerative medicine
is provided.

### Experiments and Figures

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### Example 1: Optimized method to produce human liver microtissues

**1. Thawing of cryopreserved Hepatocytes**
   - Take chosen cell-vial from cryo-tank and transfer into water bath (37 °C); set timer on 2 minutes
   - Pipette 40 ml of the Wash/Thawing medium into 50 ml tube
   - Transfer the hepatocytes into the tube, wash with 1 ml medium
   - Place 50ml tube into centrifuge; start centrifugation for 5 min at 50 rcf (corresponds to 600rpm) at RT.
   - Remove supernatant
   - Wash pellet with 20ml wash buffer
   - Place carefully 3 ml of Wash/Thawing medium into the 50ml tube
   - Re-suspend cell pellet with 2D cell culture medium
   - Count hepatocytes with e.g. Trypan Blue
**2. Hepatocyte pre-plating**
   - Use a collagen coated cell-culture dish for pre-seeding
   - Seed Hepatocytes in a 6 cm dish: 100000-250000 hepatocytes/cm²; 0.05-0.5ml per cm²
   - Place at 37°C in a CO₂ containing incubator
   - Optionally: Exchange medium after attachment with Serum-free 2D-culture medium
   - Harvest hepatocytes after 12-96 hours (daily medium exchange if longer than 12hours)
   - 3x wash with pre-warmed phosphate buffered saline the non-adherent hepatocytes away
   - Add cell detachment solution such as collagenase/accutase mixtures to culture dish to dislodge the hepatocytes from the well 50-500 ul/cm²
   - Incubate 5-30min at 37°C (visual observation until most cells are detached from the surface)
   - Carefully transfer dispersed hepatocytes into a 50ml tube prefilled with wash buffer
   - Centrifuge for 5 min with 50 rcf (corresponds to 600 rpm) at RT
   - Aspirate supernatant, add 1-50 ml wash medium to the pellet and re-suspend pellet by gentle pivoting of the tube
   - Repeat centrifugation step for 5 min with 50 rcf (corresponds to 600 rpm) at RT
   - Add 1 ml re-aggregation medium to the pellet and dissolve hepatocytes by gentle pivoting of the tube
   - Count hepatocytes
**3. Production of Microtissues**

### A. Preparation of GravityPLUS™ plates (Insphero AG, CH)

- Fill Omnitray with 15 ml of 0.75x PBS/Amphothericin and add humidifier pad
- Place frame on Omnitray
- Put lid on the plate and label plate

### B. Preparation of cell suspension

- Prepare cell suspension of defined cell number (i.e. 25'000 Heps/25'000 NPCs per ml) in falcon tubes
- Homogenize cell suspension by pivoting the tube

### C. Seeding

- Use Viaflo electronic multichannel pipette (Integra Bioscience)
- Set Viaflo parameters: Repeat dispense: 40 ul, Speed 3, Repeat: 1x (for 96-well Viaflo)
- Empty cell suspension in reservoir (don't pour more than 30 ml in the reservoir, to ensure proper distribution of cells)
- Remove lid from GravityPLUS plates
- Prior Aspiration of cell suspension, gently pivot reservoir for homogeneous distribution of cells
- Start Viaflo program: Repeat dispense
- Place Viaflo horizontally on the inserts, avoid tapping of the drop on the top by gentle pressure of the Viaflo on the inserts.
- Place lid back on plates

### 4. Media used

| **Ingredient** | **Wash buffer** | **2D cell culture medium** | **Re-aggregation medium** |
|---|---|---|---|
| Williams E Medium (GE Healthcare) | X | X | X |
| Insulin- Transferrin-Selenium (Gibco) | X | X | X |
| FBS (Fetal Calf Serum) (Lonza) | 20% | 10% | 20% |
| HGF (Hepatocyte Growth Factor) (Peprotech) | | | 20 µg/ml |

Further Ingredients in all media:
2mM Glutamine, Penecillin/Streptomycin, Amphothericin, 0.1 µM Dexamethasone

### Example 2: Stability of 3D tissue culture

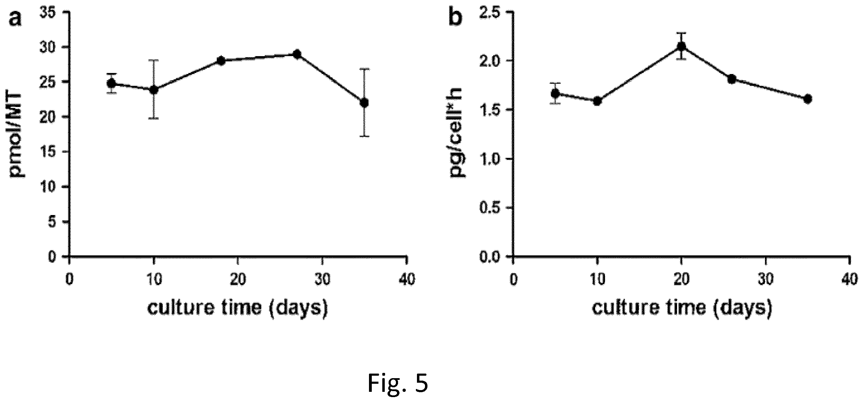

To test for the stability of 3D tissue culture obtained with the method according to the invention, Liver microtissues were monitored over time. It turned out that they remained stable over 5 weeks in culture as shown by a constant ATP content (Fig. 5a). This extended life span compared to 2D cultures of hepatocytes is most likely due to extensive cell-cell contacts, which are essential for maintaining the differentiated status of hepatocytes. Besides the stable viability, functionality of liver microtissues is preserved over 5 weeks, as indicated by persistent albumin secretion (Fig. 5b).

### Example 3: Use of 3D tissue culture produced according to the invention for hepatoxicity screening

The prolonged lifetime and functionality of the 3D tissue cultures produced according to the invention allows for long-term studies with repeated dosing to evaluate chronic hepatotoxic effects. The hepatotoxic compounds acetaminophen and diclofenac were tested with respect to their long-term toxicological profile. Acetaminophen is the major cause of drug-induced liver injury (DILI) in humans. At therapeutic doses, a proportion of the drug undergoes bio-activation by CYP2E1, CYP1A2 and CYP3A4. The reactive intermediate depletes intracellular glutathione pools leading to hepatocyte cell death. So far, 2D cultures of hepatocytes have not been able to convincingly recapitulate acetaminophen-induced toxicity in vitro (Fey and Wrzesinski 2012).

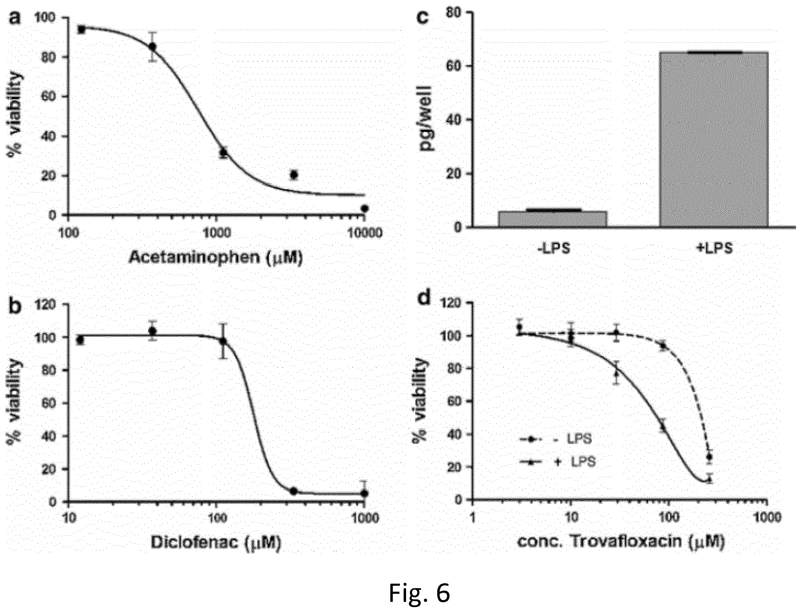

Treatment of liver microtissues over 14 days with 3 re-dosing's resulted in a concentration-dependent increasing cell death with an IC50 value of 754.2 µM (Fig. 6a). Diclofenac is a non-steroidal anti-inflammatory drug that has a strong association with hepatotoxicity. The mechanism is thought to involve phase I enzyme activity (multiple P450-catalyzed oxidations), phase II enzyme activity (glucoronylation) and mechanism-based inhibition. In comparison with 2D cultures of human hepatocytes (calculated IC50 value of 331 µM), long-term treated liver microtissues displayed an increased sensitivity toward this drug with an IC50 value of 178.6 µM (Fig. 6b). Most directly hepatotoxic compounds are detected during pre-clinical investigations. However, indirectly hepatotoxic compounds involving the immune system are not detected during pre-clinical phases, such as trovafloxacin. Recent animal experiments indicated that trovafloxacin is only hepatotoxic in combination with an inflammatory stimulus, such as lipopolysaccharide (LPS) or TNF-a. The mechanism is thought to involve enhanced cytokine secretion and accumulation in the liver, causing caspase activation and subsequent liver injury. Induction of the inflammatory response in liver microtissues by LPS resulted in elevated levels of IL-6 secretion, verifying the responsiveness of incorporated macrophages in the liver microtissues (Fig. 6c). The addition of LPS shifted the hepatotoxic threshold of trovafloxacin about threefold from 220 (without LPS) to 71 µM in the presence of LPS (Fig. 6d). Developed to overcome the limitations of conventional 2D culture, multi-cell type 3D liver microtissues resemble liver-like cell composition and an extended stability in culture. The long-term viability and functionality of liver microtissues allows for routine compound testing as well as chronic and inflammation-mediated toxicity. The 96-well format allows for microtissue mass production enabling the implementation of an organotypic liver model at an early time point in drug development.

### Figures

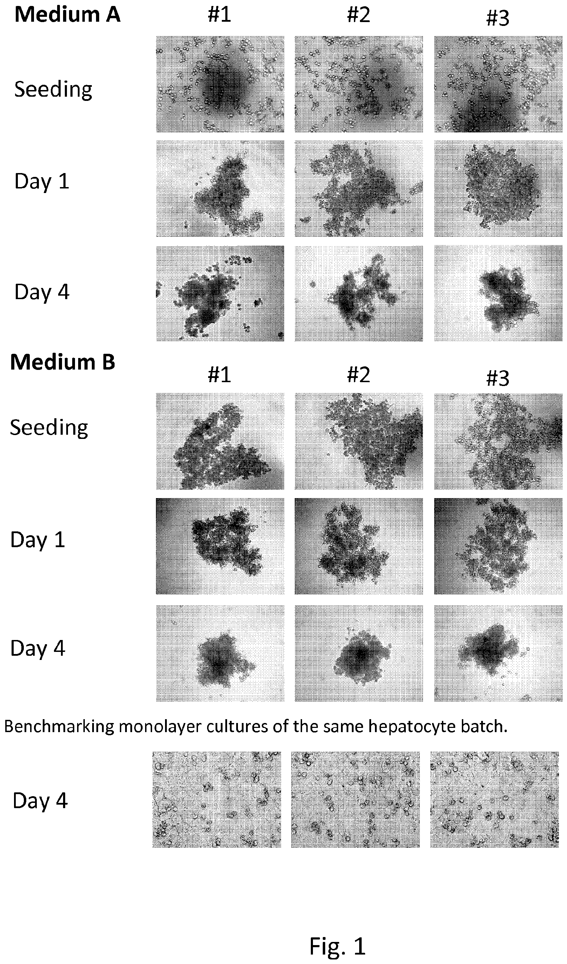

**Fig. 1** **Incomplete microtissue formation if seeded directly after thawing**
   Human liver microtissue formation was initiated with cryopreserved, plateable primary human hepatocytes, which were seeded directly after thawing in hanging drop plates. Two different medium compositions were tested (Medium A + B). Three representative hanging drops (Nr.1, 2, 3) were imaged directly after seeding and after 1 and 4 days in hanging drops. The hepatocytes accumulated at the meniscus of the in hanging drop and formed loose aggregates until day 4 in culture. The same hepatocyte lot displayed attachment to 2D collagen-I coated culture dishes after thawing, showing the suitability of this hepatocyte for 2D culture.

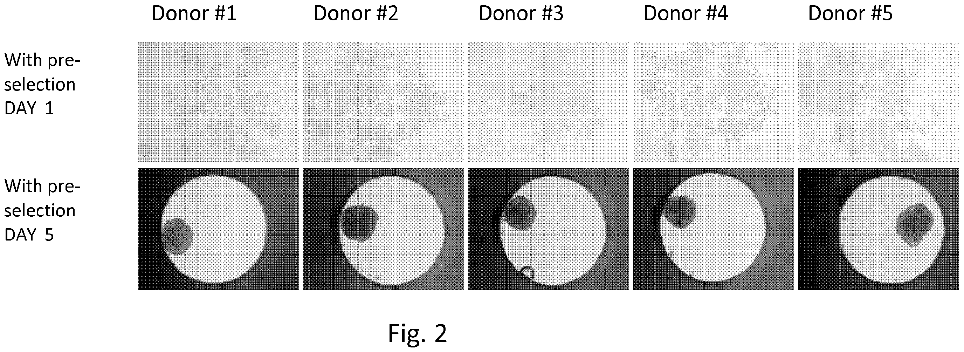

**Fig. 2** **Vital cell selection allows for complete microtissue formation**
   Human liver microtissue formation was initiated with 5 independent lots (donor 1-5) of cryopreserved, plateable primary human hepatocytes, which were seeded directly after thawing on collagen-I coated 2D culture dishes for 24 hours. After vital cell selection, the cells were detached from the culture dish and seeded in hanging drop plates. The hepatocytes accumulated at the meniscus of the in hanging drop and formed tight microtissues (also called "hepatospheres") within 5 days of culture. The microtissues were transferred to a receiver plate (GravityTRAP™) and imaged for microtissue appearance and -size. All 5 hepatocyte lots showed robust and uniform microtissue formation within 5 days of culture.

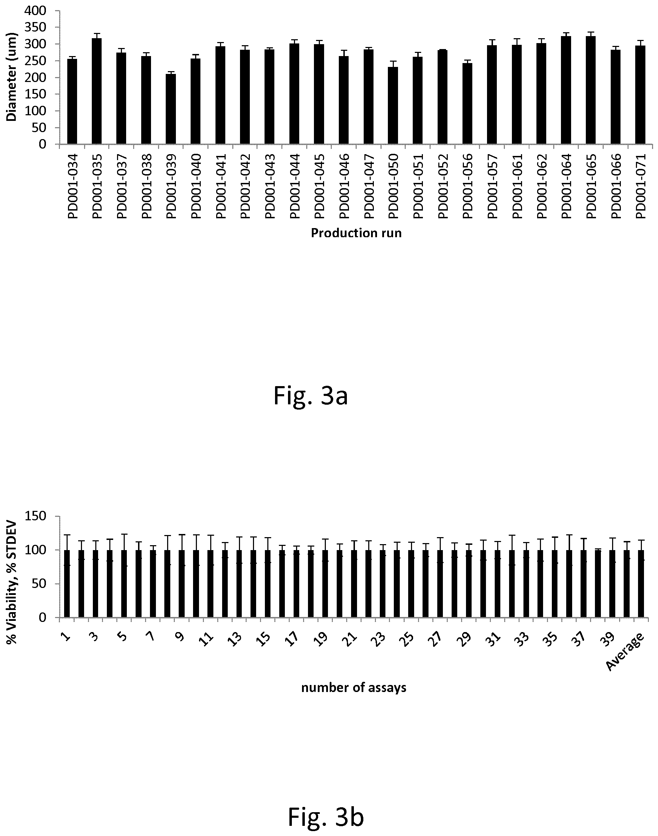

**Fig. 3** **Reproducible formation of human liver microtissues with vital cell selection of primary human hepatocytes**
   (A) Size variation of human liver microtissues produced after pre-plating of cryopreserved human hepatocytes. The diameter human liver microtissues of 24 production runs is shown, including the standard deviation (n=6). The diameter of human liver microtissues of 24 production runs with the same hepatocyte lot was determined. The average diameter and standard deviation of the size measurement of 6 microtissues per run is shown. The microtissues showed less than 5% size variation within each production run. The average microtissue size of all 24 productions was 280 µm, with an relative size deviation of less than 10% between production runs.
   (B) Resulting assay variability. The intracellular ATP-content of 40 assays with human liver microtissues was quantified with CellTiter-Glo®-assay after 14 days. Measurements were performed in triplicates. The average RLU's from all 40 assays was set to 100%, the relative standard deviation of each measurement to the mean is depicted. Average relative standard deviation of all 40 measurements is 14.6%.

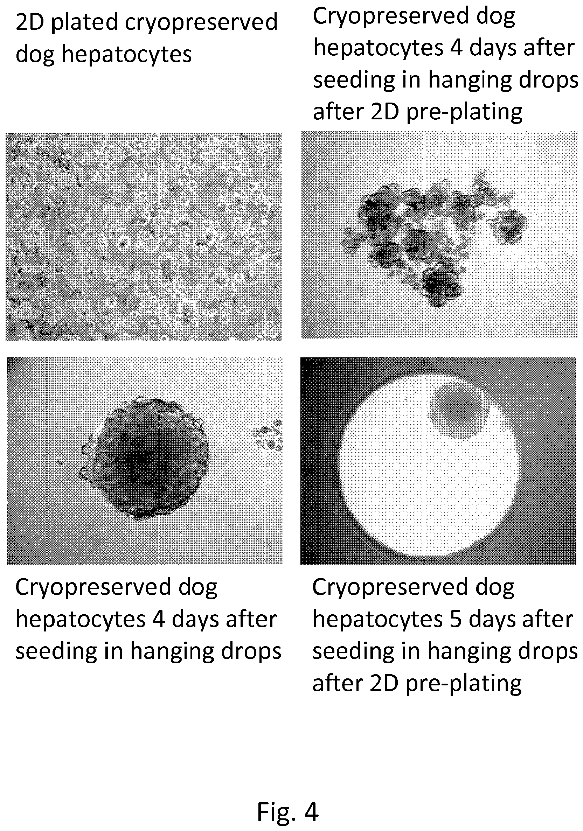

**Fig. 4** **Microtissue formation of dog hepatocytes is achieved only with vital cell selection**
   Dog liver microtissue formation was initiated with cryopreserved, plateable primary dog hepatocytes, which were seeded either directly in the hanging drops (B) or pre-plated on collagen-I coated 2D culture dishes for 24 hours (C, D). Dog liver microtissue formation was not observed after direct seeding of cryopreserved hepatocytes until 4 days in culture. Vital-cell selected dog hepatocytes formed dense microtissues within 4 days in hanging drops (C). (D) Image of a dog liver microtissue transferred from the hanging-drop to the receiver plate.
**Fig. 5** **Viability and functionality of 3D tissues over 5 weeks in culture.**
   (A) Intra-tissue ATP quantification. ATP content per microtissue is depicted (pmol ATP/MT) as an indicator of cell viability and vitality.
   (B) Quantification of secreted albumin by ELISA over time, normalized to the initial hepatocyte cell number and time
**Fig. 6** **Long-term toxicity and inflammation-mediated testing with human liver microtissues produced according to the invention**
   (A) Dose-response of acetaminophen toxicity after 14 days treatment (3 re-dosing) resulted in an IC5₀ value of 754.2 µM
   (B) Dose-response curve of diclofenac supplemented for 14 days (3 re-dosing) resulted in an IC5₀ value of 178.6 µM.
   (C) Quantification of IL-6 secretion with ELISA measurement. Hepatosphere was induced for 48 h with 10 lg/ml LPS. Induction with LPS led to a tenfold increase in IL-6 secretion.
   (D) Dose-response of trovafloxacin induced toxicity in presence and absence of LPS. Presence of LPS decreased the IC50 threefold from 220 (-LPS) to 71 µM (+ LPS)

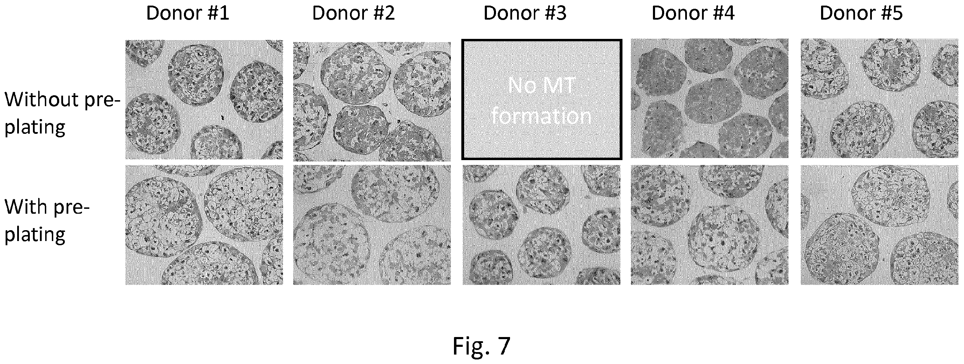

**Fig. 7** **Structural integrity of human liver microtissues produced without pre-plating and with pre-plating**

Histological preparations were made from formalin fixed and paraffin embedded human liver microtissues produced either directly after thawing of the cryopreserved hepatocytes or after pre-plating of the hepatocytes for 24h on a collagen-coated cell culture dish. 3-5um thick sections were stained with Hematoxylin and eosin (H&E). Images were taken with a 10x objective.

The upper row of Fig. 7 shows microtissues which have been created without prior pre-plating, while the low reo shows microtissues which have been created with prior pre-plating.

For donor #3 no microtissues were formed using the cryopreserved hepatocytes without the pre-plating step. For the other preparations, a high degree of necrotic areas apparent by dense eosinophilic staining and lack of nuclei within the tissues were visible in the groups without pre-plating (especially predominant for donor 2 and 4 which appear to have hardly any viable hepatocytes integrated). The pre-plating step significantly improves structural morphology of the liver microtissues.

### References

Pichard et al. (2006) Human hepatocyte culture. Meth Mol Biol 320: 283-293.
Kelm et al., Method for generation of homogeneous multicellular tumor spheroids applicable to a wide variety of cell types. Biotechnol. Bioeng. 2003, 83, 173-180.
Kelm et al 2010, A novel concept for scaffold-free vessel tissue engineering: self-assembly of microtissue building blocks. Journal of Biotechnology
Ridky TW et al., Invasive three-dimensional organotypic neoplasia from multiple normal human epithelia. Nature Medicine 2010
Cody NA et al., Influence of monolayer, spheroid, and tumor growth conditions on chromosome 3 gene expression in tumorigenic epithelial ovarian cancer cell lines. BMC Med Genomics. 2008
Kelm & Fussenegger, Microscale tissue engineering using gravity-enforced cell assembly. Trends Biotechnol. 2004, 22, 195-202.
Yuhas, J et al., Simplified method for production and growth of multicellular tumor spheroids. Cancer Res. 1977, 37, 3639-3643.
Metzger et al, The liquid overlay technique is the key to formation of co-culture spheroids consisting of primary osteoblasts, fibroblasts and endothelial cells. Cytotherapy. 2011 Sep;13(8):1000-12.
Geckil et al, Engineering hydrogels as extracellular matrix mimics. Nanomedicine (Lond). 2010 April; 5(3): 469-484.
Carletti E et al, Scaffolds for tissue engineering and 3D cell culture. Methods Mol Biol. 2011;695:17-39.
Fey and Wrzesinski, Determination of drug toxicity using 3D spheroids constructed from an immortal human hepatocyte cell line. Toxicol Sci. 2012 Jun;127(2):403-11
Jensen J, Hyllner J, Bjorquist P. Human embryonic stem cell technologies and drug discovery. J Cell Physiol 2009; 219: 513-519.
Sartipy & Björquist. Concise Review: Human Pluripotent Stem Cell-Based Models for Cardiac and Hepatic Toxicity Assessment. Stem Cells 2011; 29 (5): 744-748

## Claims

1. A method of preparing cells for 3D tissue culture, the method comprising the steps of
a) plating hepatocytes on a suitable surface,
b) optionally, checking for their capability to adhere to said surface,
c) discarding the cells which have not adhered to said surface,
d) detaching the adhered cells and transferring them into a 3D tissue culture process,
wherein the method does not encompass the application of a viability assay throughout steps a) - d),
in which method the cells are not expanded any time prior to, during or after plating, and the cells are not passaged after plating.

2. The method according to claim 1, in which method the 3D tissue culture process is at least one selected from the group consisting of
• scaffold-based 3D tissue culture
• hydrogel-based 3D tissue culture
• cellular self-assembly 3D tissue culture, and/or
• 3D bioprinting.

3. The method according to any of the preceding claims, in which method the cells are cryopreserved cells which are thawed before plating.

4. The method according to claims 1 or 2, in which method the cells are non-frozen cells.

5. The method according to any of the preceding claims, in which method the cells are mammalian cells.

6. The method according to claim 5, wherein the cells are
• human cells
• cynomolgus cells
• pig cells
• canine cells, or
• rat cells.

7. The method according to any of the preceding claims, wherein the 3D tissue culture adopts the shape of a spheroid.

8. The method according to any of the preceding claims, wherein the 3D tissue culture is used for at least one purpose selected from the group consisting of:
• drug efficacy and/or toxicity screenings,
• investigative/mechanistic toxicology,
• target discovery/identification,
• drug repositioning studies,
• pharmacokinetics and pharmacodynamics assays, and/or
• regenerative medicine.

## Patentansprüche

1. Verfahren zur Vorbereitung von Zellen für eine 3D-Gewebekultur, wobei das Verfahren die Schritte aufweist
a) Hepatozyten auf eine geeignete Oberfläche ausplattieren,
b) optional ihre Fähigkeit überprüfen, an besagter Oberfläche zu haften,
c) Zellen verwerfen, die nicht an der Oberfläche haften,
d) adhärente Zellen ablösen und sie in einen 3D-Gewebekulturprozess überführen,
wobei das Verfahren nicht die Anwendung eines Lebensfähigkeitstests während der Schritte a) - d) aufweist,
wobei bei diesem Verfahren die Zellen zu keinem Zeitpunkt vor, während oder nach dem Plattieren expandiert werden, und die Zellen nach dem Ausplattieren nicht passagiert werden.

2. Verfahren nach Anspruch 1, wobei bei dem Verfahren der 3D-Gewebekulturprozess mindestens einer ausgewählt aus der Gruppe ist, die aus
• 3D-Gewebekultur auf Gerüstbasis
• 3D-Gewebekultur auf Hydrogelbasis
• zellulärer sich selbst-aufbauender 3D-Gewebekultur und/oder
• 3D-Bioprinting
besteht.

3. Verfahren nach einem der vorhergehenden Ansprüche, bei dem die Zellen kryokonservierte Zellen sind, die vor dem Ausplattieren aufgetaut werden.

4. Verfahren nach Anspruch 1 oder 2, wobei es sich bei den Zellen um nicht eingefrorene Zellen handelt.

5. Verfahren nach einem der vorhergehenden Ansprüche, wobei es sich bei den Zellen um Säugetierzellen handelt.

6. Verfahren nach Anspruch 5, wobei es sich bei den Zellen um
• menschliche Zellen
• Cynomolgus-Zellen
• Schweinezellen
• Hundezellen oder
• Rattenzellen
handelt.

7. Verfahren nach einem der vorhergehenden Ansprüche, wobei die 3D-Gewebekultur die Form eines Sphäroids annimmt.

8. Verfahren nach einem der vorhergehenden Ansprüche, wobei die 3D-Gewebekultur für mindestens einen Zweck verwendet wird, ausgewählt aus der Gruppe bestehend aus:
• Wirksamkeits- und/oder Toxizitätsdurchmusterungen von Arzneimitteln,
• investigative/mechanistische Toxikologie,
• Target-Entdeckung/-identifizierung,
• Studien zur Neupositionierung von Arzneimitteln,
• Pharmakokinetik- und Pharmakodynamik-Untersuchungen und/oder
• regenerative Medizin.

## Revendications

1. Procédé de préparation de cellules pour une culture de tissu en 3D, le procédé comprenant les étapes de
a) placage de cellules hépatiques sur une surface appropriée,
b) de manière facultative, vérification de leur capacité à adhérer à ladite surface,
c) rejet des cellules qui n'ont pas adhéré à ladite surface,
d) détachement des cellules ayant adhéré et leur transfert jusque dans un processus de culture de tissu en 3D, dans lequel le procédé n'englobe pas l'application d'un dosage de viabilité tout au long des étapes a) à d),
procédé dans lequel les cellules ne sont pas étendues à n'importe quel moment avant, pendant ou après placage, et les cellules ne sont pas en transit après placage.

2. Procédé selon la revendication 1, procédé dans lequel le processus de culture de tissu en 3D est au moins un processus sélectionné dans le groupe constitué par
• une culture de tissu en 3D à base d'échafaudage
• une culture de tissu en 3D à base d'hydrogel
• une culture de tissu en 3D à auto-assemblage cellulaire, et/ou
• une bio-impression 3D.

3. Procédé selon l'une quelconque des revendications précédentes, procédé dans lequel les cellules sont des cellules conservées par cryogénisation qui sont dégelées avant placage.

4. Procédé selon les revendications 1 ou 2, procédé dans lequel les cellules sont des cellules non gelées.

5. Procédé selon l'une quelconque des revendications précédentes, procédé dans lequel les cellules sont des cellules mammifères.

6. Procédé selon la revendication 5, dans lequel les cellules sont
• des cellules humaines
• des cellules de macaque de Buffon
• des cellules de porc
• des cellules canines, ou
• des cellules de rat.

7. Procédé selon l'une quelconque des revendications précédentes, dans lequel la culture de tissu en 3D adopte la forme d'un sphéroïde.

8. Procédé selon l'une quelconque des revendications précédentes, dans lequel la culture de tissu en 3D est utilisée pour au moins un but sélectionné dans le groupe constitué par :
• examens de l'efficacité et/ou de la toxicité d'un médicament,
• toxicologie investigatrice/mécaniste,
• découverte/identification de cible,
• études de repositionnement de médicament,
• dosages pharmacocinétiques et pharmacodynamiques, et/ou
• médecine régénératrice.
